# FASCICULE DE BREVET EUROPEEN

(11) **EP 2 954 213 B1**
(45) Date de publication et mention de la délivrance du brevet: **08.02.2017**
(21) Numéro de dépôt: 13818765.3
(22) Date de dépôt: 18.12.2013
(51) Int. Cl.: F04D 29/42, F04D 29/66, A61M 16/00

(54) **TURBINE POUR APPAREIL D'ASSISTANCE RESPIRATOIRE A ETANCHEITE GAZEUSE AMELIOREE**
TURBINE FÜR EINE BEATMUNGSHILFSVORRICHTUNG MIT VERBESSERTER GASDICHTIGKEIT
TURBINE FOR A BREATHING ASSISTANCE APPARATUS WITH IMPROVED GAS TIGHTNESS

(30) Priorité: 11.02.2013 FR 1351123
(43) Date de publication de la demande: 16.12.2015
(73) Titulaire: Air Liquide Medical Systems, 92160 Antony (FR)
(72) Inventeur: DUBOIS, Pierre-Emmanuel, F-92330 Sceaux (FR)
(74) Mandataire: Pittis, Olivier
(86) Numéro de dépôt international: PCT/FR2013/053158
(87) Numéro de publication internationale: WO 2014/122368

(56) Documents cités:
- FR-A1- 2 289 776
- FR-A1- 2 908 482
- US-A- 3 485 443
- US-A1- 2012 199 129

## Description

L'invention concerne une micro-soufflante ou turbine destinée à équiper un appareil d'assistance respiratoire. Plus particulièrement, l'invention porte sur l'amélioration de l'étanchéité de la partie aéraulique, c'est-à-dire de la volute.

Certains appareils d'assistance respiratoire mettent en oeuvre des micro-soufflantes ou turbines servant à générer un gaz sous pression, en général de l'air ou de l'air enrichi en oxygène, qui est ensuite envoyé vers les voies aériennes d'un patient.

Une turbine de ce type est notamment décrite par le document EP-A-2102504 qui enseigne un appareil d'assistance respiratoire permettant une délivrance régulée d'un gaz, notamment d'air, au moyen d'une turbine comprenant un boîtier, un conduit d'amenée d'air délimité par le boîtier, une volute dont l'ouverture d'entrée est en communication avec le conduit d'amenée d'air, une roue à ailettes, située immédiatement en aval de l'ouverture d'entrée de la volute, et un moteur d'entraînement de la roue en rotation de manière à générer un flux d'air centrifuge dans la volute.

La volute est donc le compartiment au sein duquel est aménagé la roue à ailettes servant à générer le flux gazeux envoyé au patient. Pour des raisons de production, il est aujourd'hui très difficile, voire quasi-impossible de réaliser l'ensemble de la forme de la volute en une seule et même pièce.

La volute de la turbine est donc généralement formée de deux pièces venant se coupler l'une à l'autre, à savoir une partie supérieure de volute, encore appelée volute supérieure, et une partie inférieure de volute, encore appelée volute inférieure.

La solidarisation des deux parties de volutes est obtenue par collage des deux volutes l'une avec l'autre.

Dans le cas de l'utilisation d'une roue sans flasque, pour garantir les performances de la micro-soufflante, la volute supérieure doit être positionnée précisément par rapport à la roue à ailettes. Or, le grand nombre de pièces mises en jeu dans l'assemblage crée une chaîne de cotes ne permettant pas de positionner précisément la volute inférieure par rapport à la roue, et un jeu non maîtrisé est donc créé entre les deux parties de volutes, lors de leur couplage.

Le collage des deux parties de volutes l'une avec l'autre ne permet pas de combler parfaitement ce jeu et il en résulte des problèmes d'étanchéité. En d'autres termes, le jeu non maîtrisé existant entre les deux parties de volute, après leur collage l'une avec l'autre, engendre des problèmes de fuites et d'étanchéité gazeuse.

Le problème qui se pose dès lors est de réaliser une étanchéité améliorée entre les parties de volute alors qu'on ne maîtrise pas l'espacement entre ces parties, de manière à supprimer ou minimiser les problèmes de fuites et d'étanchéité gazeuse.

La solution est alors une turbine pour appareil d'assistance respiratoire comprenant un moteur électrique coopérant avec une roue à ailettes pour générer un flux de gaz, et une volute comprenant une partie inférieure de volute et une partie supérieure de volute avec un passage d'entrée de gaz, venant se coupler l'une à l'autre et conformées de manière à former un compartiment interne dans lequel est agencée la roue à ailettes, et
dans laquelle la partie inférieure de volute comprend un premier rebord périphérique et la partie supérieure de volute comprend un second rebord périphérique, lesdits premier rebord périphérique et second rebord périphérique se faisant face lorsque la partie inférieure de volute est couplée à la partie supérieure de volute, caractérisée en ce qu'au moins une structure en matériau élastomère est agencée, en particulier montée serrée, entre ou autour d'au moins une partie des rebords périphériques des deux parties de volute, la structure en matériau élastomère comprenant une première portion de forme annulaire ou semi-annulaire, et la structure en matériau élastomère comprenant une seconde portion de forme tubulaire comprenant un passage central.

En fait, la structure en matériau élastomère agencée le long des rebords périphériques des deux parties de volute permet non seulement un maintien mécanique et un bon positionnement des volutes mais aussi une bonne étanchéité gazeuse, y compris au niveau du conduit d'évacuation de gaz de la turbine.

Selon le cas, la turbine de l'invention peut comprendre l'une ou plusieurs des caractéristiques techniques suivantes :
- le matériau élastomère est un caoutchouc, un silicone ou un élastomère thermo-plastique.
- la seconde portion de forme tubulaire vient se raccorder à la première portion de forme annulaire ou semi-annulaire.
- la partie inférieure de volute et la partie supérieure de volute sont conformées pour définir au moins une partie d'un conduit d'évacuation servant à évacuer le gaz débité par la roue à ailettes au sein du compartiment comprenant ladite roue à ailettes, la seconde portion de forme tubulaire venant se positionner en continuité dudit conduit d'évacuation de gaz.
- la structure en matériau élastomère, la partie inférieure de volute et la partie supérieure de volute sont maintenues solidaires par collage ou par la pièce élastomère elle-même.
- la partie inférieure de volute et la partie supérieure de volute sont en matériau polymère, en particulier en thermo plastique, par exemple de type PC, ABS, ABS/PC...
- le moteur permet d'entrainer la roue à ailettes à une vitesse comprise entre 0 et 100 000 tr/min, typiquement entre 10 000 et 60 000 tr/min.
- la première portion de forme annulaire ou semi-annulaire, et la seconde portion de forme tubulaire sont formées d'une pièce unique.
- la première portion de forme annulaire ou semi-annulaire, et la seconde portion de forme tubulaire forment une pièce unique obtenue par moulage.

L'invention porte aussi sur un appareil d'assistance respiratoire comprenant une turbine selon l'invention.

La présente invention va maintenant être décrite plus en détail en références aux Figures annexées parmi lesquelles :
- les Figures 1 et 2 représentent une vue schématique (de côté) d'un mode de réalisation d'une micro-soufflante ou turbine pour appareil d'assistance respiratoire selon l'invention, avant mise en place de la structure en matériau élastomère ;
- la Figure 3 montre une vue schématique (de côté) d'un mode de réalisation d'une micro-soufflante ou turbine pour appareil d'assistance respiratoire, après mise en place de la structure en matériau élastomère selon la présente invention ; et
- la Figure 4 représente un mode de réalisation de la structure en matériau élastomère selon la présente invention.

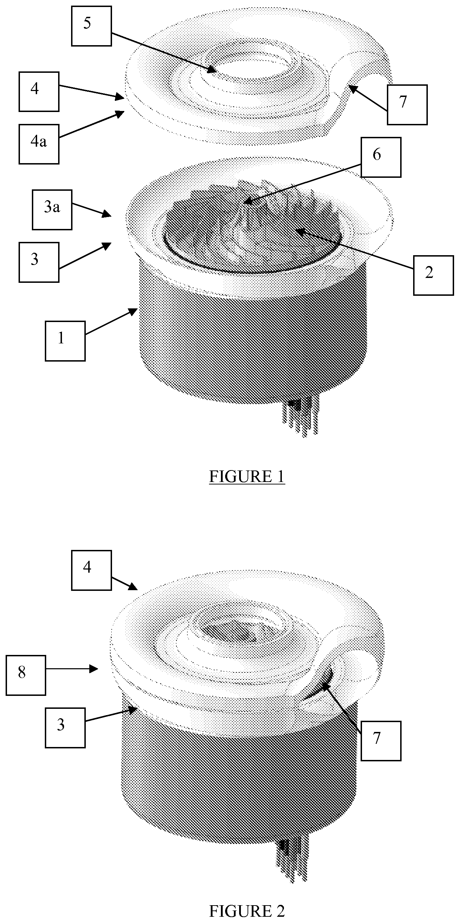

Les Figures 1 et 2 schématisent un mode de réalisation d'une micro-soufflante ou turbine motorisée équipant un appareil d'assistance respiratoire comprenant un moteur électrique 1, situé dans un capotage, entrainant, via son axe rotatif 6, une roue à ailettes 2 servant à générer un flux de gaz, typiquement un flux d'air.

La roue à ailettes 2 est située dans une structure tridimensionnelle 3, 4 appelée « volute » comprenant une partie inférieure de volute 3, couramment appelée volute inférieure, et une partie supérieure de volute 4, appelée volute supérieure, venant se raccorder l'une à l'autre.

Les parties inférieure et supérieure de volute 3, 4 définissent ainsi entre elles un espace interne ou compartiment interne englobant la roue à ailettes 2, c'est-à-dire que la roue à ailettes 2 est prise en sandwich dans le compartiment interne formé entre les parties inférieure et supérieure de volute 3,4.

Les parties inférieure et supérieure de volute 3, 4 sont également conformées pour définir au moins une partie d'un conduit d'évacuation 7 servant à l'acheminement du gaz débité par ladite roue à ailettes 2.

L'air est aspiré par la roue à ailettes 2 au travers une ouverture d'entrée 5 située au centre de la partie supérieure de volute 4 par laquelle transite l'air aspiré par la roue à ailettes 2 qui est située immédiatement en aval de l'ouverture d'entrée 5 de la volute. Toutefois, de façon alternative, l'entrée de l'air peut aussi se faire au travers d'une ouverture d'entrée située latéralement dans la volute 4.

L'air est évacué ensuite par la roue à ailettes 2, via le conduit d'évacuation 7 de gaz, sous forme d'un flux d'air centrifuge engendré par la rotation de la roue à ailettes 2 lorsqu'elle est entraînée par le moteur 1, puis le gaz est véhiculé jusqu'au patient.

Les parties supérieure 4 et inférieure 3 de volute sont de section générale en spirale, c'est-à-dire que leur forme évolue de manière régulière et constamment positive (convexe).

La partie inférieure de volute 3 a une forme générale de coupelle dont le fond comporte une ouverture centrale venant former manchon autour d'une partie de la paroi ou capotage externe du moteur 1 ou d'un compartiment renfermant le moteur 1, lorsque la partie inférieure de volute 3 est positionnée autour dudit moteur 1, comme illustré sur la Figure 1. Un joint d'étanchéité (non visible) permet d'assurer une étanchéité gazeuse entre le fond de la partie inférieure de volute 3 et la paroi ou capotage externe du moteur 1 ou du compartiment renfermant le moteur 1.

Sur la Figure 1, la partie supérieure de volute 4 a été découplée de la partie inférieure de volute 3 pour permettre de visualiser le compartiment interne comprenant la turbine 2 et son axe 6 d'entraînement en rotation.

A l'inverse, sur la Figure 2, la partie supérieure de volute 4 est représentée en position couplée sur la partie inférieure de volute 3, c'est-à-dire que les deux parties de volute 3, 4 sont en contact l'une avec l'autre le long d'une zone de jonction 8 quasi-circulaire située au niveau des rebords périphériques 3a, 4a desdites parties de volute 3, 4 qui viennent en contact l'un avec l'autre.

En d'autres termes, la zone de jonction 8 définie par les rebords 3a, 4a desdites parties de volute 3, 4, en contact l'un avec l'autre, forme une quasi-couronne qui est interrompue au niveau du conduit d'évacuation 7 de gaz.

La turbine des Figures 1 et 2 est donc représentée avant mise en place de la structure en matériau élastomère.

Afin de solidariser les deux parties de volute 3, 4 l'une à l'autre, il est habituel de les coller ensemble le long de cette zone de jonction 8, c'est-à-dire de coller ensemble les rebords périphériques 3a, 4a, mais cela ne produit pas une liaison étanche car il existe toujours un jeu non maîtrisé existant entre les rebords périphériques 3a, 4a des deux parties de volute, après leur collage l'une avec l'autre, lequel jeu engendre des problèmes de fuites et d'étanchéité gazeuse.

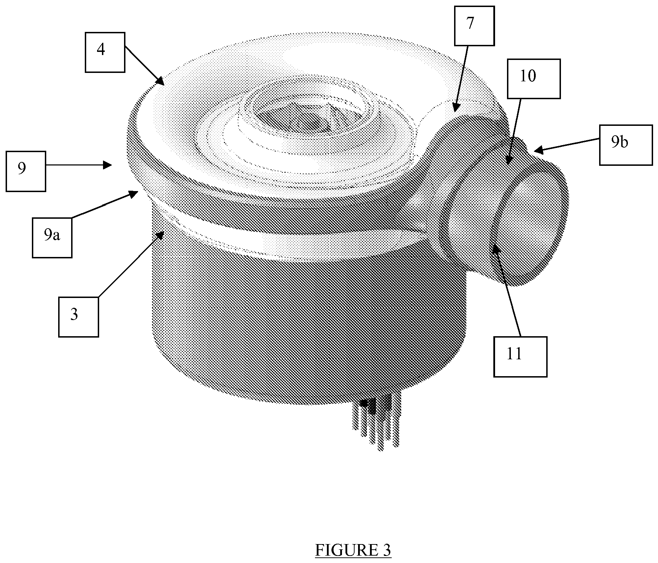

Afin d'y remédier, comme illustré en Figure 3, conformément à la présente invention, une pièce 9 en élastomère de forme annulaire ou semi-annulaire est insérée entre les rebords périphériques 3a, 4a des deux parties de volute 3, 4.

Le matériau élastomère est choisi de sorte de satisfaire notamment aux critères suivants:
- une souplesse adaptée permettant une déformation suffisante autorisant sa mise en forme par allongement,
- une résistance à la température due à échauffement aéraulique, et
- une conservation des caractéristiques mécaniques d'élasticité au cours du temps du fait que la pièce est soumise à un allongement constant.

Par exemple, en guise de matériau élastomère, on peut choisir le caoutchouc, le silicone, un élastomère thermoplastique TPE...

En fait, la pièce 9 en élastomère enserre les deux volutes 3, 4. La mise sous tension de la pièce élastomère 9 assure ainsi un serrage naturel sur l'ensemble composé des volutes inférieure 3 et supérieure 4 au niveau des rebords périphériques 3A, 4A. On utilise les caractéristiques d'élasticité du matériau afin d'assurer étanchéité et maintien en position.

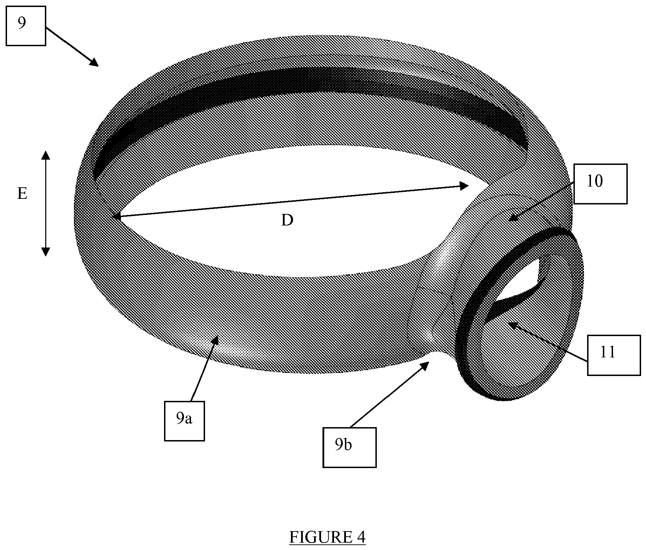

Selon un mode de réalisation illustré sur la Figure 4, la pièce ou structure 9 en élastomère comprend une première portion 9a en forme générale de couronne ou de quasi-couronne, c'est-à-dire annulaire ou semi-annulaire. Typiquement, cette portion 9a en couronne ou quasi-couronne à une épaisseur E comprise entre 0,5 et 3 mm, une largeur L comprise entre 4 et 20 mm, et un diamètre D compris entre 45 et 100 mm.

Par ailleurs, la pièce ou structure 9 en élastomère comprend une seconde portion 9b, venant se raccorder à la première portion 9a, de préférence ces portions 9a, 9b sont formées d'une pièce unique, par exemple obtenue par moulage ou analogue.

La seconde portion 9b est de forme tubulaire 10 et comprend un passage central 11 apte à véhiculer le gaz provenant du compartiment où se trouve la turbine 2.

En fait, cette seconde portion 9b de forme tubulaire 10 vient se positionner, au moins partiellement, en prolongement du conduit d'évacuation de gaz 7 recevant le flux d'air créé par la roue à ailettes 2, comme représenté sur la Figure 3. Dit autrement, la paroi du conduit d'évacuation 7 est prolongée par la seconde portion 9b de forme tubulaire 10 de la pièce ou structure 9 en élastomère.

L'entrée du conduit 9b est comprimée contre la sortie du conduit 7 car le conduit 9b forme une seule pièce avec l'élastomère 9a resserrée autours de l'ensemble volute 3, 4.
C'est donc le même principe qui assure l'étanchéité entre les deux volutes et entre les conduits 7 et 9b.

Le gaz, c'est-à-dire en général de l'air, accéléré par la roue à ailettes 2 est récupéré par le conduit d'acheminement 7 aménagé dans les deux parties de volute 3, 4 comme visible sur les Figures, et la portion 9b tubulaire de la pièce ou structure 9 en élastomère, puis est envoyé vers un circuit respiratoire relié à un patient.

Une turbine selon l'invention peut servir à traiter des pathologies respiratoires de tout type, en particulier apnée du sommeil, BPCO, SLA, troubles liés à obésité...

Typiquement une turbine selon l'invention est aménagée dans un appareil d'assistance respiratoire de type CPAP (à pression continue) ou BiPAP (à deux niveaux de pression), lequel alimente un patient en gaz respiratoire par l'intermédiaire d'un conduit flexible d'acheminement du gaz et d'une interface patient, tel un masque respiratoire ou des lunettes nasales.

## Revendications

1. Turbine pour appareil d'assistance respiratoire comprenant :
- un moteur électrique (1) coopérant avec une roue à ailettes (2) pour générer un flux de gaz, et
- une volute (3, 4) comprenant une partie inférieure de volute (3) et une partie supérieure de volute (4) avec un passage d'entrée de gaz (5), venant se coupler l'une à l'autre et conformées de manière à former un compartiment interne dans lequel est agencée la roue à ailettes (2),
et dans laquelle la partie inférieure de volute (3) comprend un premier rebord périphérique (3a) et la partie supérieure de volute (4) comprend un second rebord périphérique (4a), lesdits premier rebord périphérique (3a) et second rebord périphérique (4a) se faisant face lorsque la partie inférieure de volute (3) est couplée à la partie supérieure de volute (4),
**caractérisée en ce qu'**au moins une structure (9) en matériau élastomère est agencée entre ou autour d'au moins une partie des rebords périphériques (3a, 4a) des deux parties de volute (3, 4), la structure (9) en matériau élastomère comprenant une première portion (9a) de forme annulaire ou semi-annulaire, et une seconde portion (9b) de forme tubulaire (10) comprenant un passage central (11).

2. Turbine selon la revendication précédente, **caractérisée en ce que** le matériau élastomère est un caoutchouc, un silicone ou un élastomère thermo-plastique.

3. Turbine selon l'une des revendications précédentes, **caractérisée en ce que** la seconde portion (9b) de forme tubulaire (10) vient se raccorder à la première portion (9a) de forme annulaire ou semi-annulaire.

4. Turbine selon l'une des revendications précédentes, **caractérisée en ce que** la première portion (9a) de forme annulaire ou semi-annulaire, et la seconde portion (9b) de forme tubulaire (10) sont formées d'une pièce unique.

5. Turbine selon l'une des revendications précédentes, **caractérisée en ce que** la première portion (9a) de forme annulaire ou semi-annulaire, et la seconde portion (9b) de forme tubulaire (10) forment une pièce unique obtenue par moulage.

6. Turbine selon l'une des revendications précédentes, **caractérisée en ce que** la partie inférieure de volute (3) et la partie supérieure de volute (4) sont conformées pour définir au moins une partie d'un conduit d'évacuation (7) servant à évacuer le gaz débité par la roue à ailettes (2) au sein du compartiment comprenant ladite roue à ailettes (2), la seconde portion (9b) de forme tubulaire (10) venant se positionner en continuité dudit conduit d'évacuation (7) de gaz.

7. Turbine selon l'une des revendications précédentes, **caractérisée en ce que** la structure (9) en matériau élastomère, la partie inférieure de volute (3) et la partie supérieure de volute (4) sont solidaires.

8. Turbine selon l'une des revendications précédentes, **caractérisée en ce que** la partie inférieure de volute (3) et la partie supérieure de volute (4) sont en matériau polymère, en particulier en thermo plastique.

9. Turbine selon l'une des revendications précédentes, **caractérisée en ce que** la partie inférieure de volute (3) et la partie supérieure de volute (4) sont en matériau thermo plastique.

10. Turbine selon l'une des revendications précédentes, **caractérisée en ce que** le moteur permet d'entrainer la roue à ailettes (2) à une vitesse comprise entre 0 et 100 000 tr/min, typiquement entre 10 000 et 60 000 tr/min.

11. Appareil d'assistance respiratoire comprenant une turbine selon l'une des revendications précédentes.

## Patentansprüche

1. Turbine für ein Gerät zur Atmungsunterstützung, Folgendes umfassend:
- einen Elektromotor (1), der mit einem Flügelrad (2) zusammenwirkt, um einen Gasstrom zu erzeugen, und
- ein Spiralgehäuse (3, 4), das einen unteren Spiralgehäuseabschnitt (3) und einen oberen Spiralgehäuseabschnitt (4) mit einem Gaseinlasskanal (5) umfasst, die sich miteinander koppeln und ausgebildet sind, um eine innere Kammer zu bilden, in der das Flügelrad (2) angeordnet ist,
und wobei der untere Spiralgehäuseabschnitt (3) einen ersten umlaufenden Rand (3a) umfasst und der obere Spiralgehäuseabschnitt (4) einen zweiten umlaufenden Rand (4a) umfasst, wobei der erste umlaufende Rand (3a) und der zweite umlaufende Rand (4a) einander gegenüberliegen, wenn der untere Spiralgehäuseabschnitt (3) mit dem oberen Spiralgehäuseabschnitt (4) gekoppelt ist,
**dadurch gekennzeichnet, dass** zumindest eine Struktur (9) aus Elastomer-Material zwischen oder um zumindest einen Teil der umlaufenden Ränder (3a, 4a) der beiden Spiralgehäuseabschnitte (3, 4) angeordnet ist, wobei die Struktur (9) aus Elastomer-Material einen ersten Abschnitt (9a) in Form eines Ringes oder eines Halbringes umfasst, und einen zweiten Abschnitt (9b) in Röhrenform (10), einen zentralen Durchlass (11) umfassend.

2. Turbine nach dem vorherigen Anspruch, **dadurch gekennzeichnet, dass** Elastomer-Material ein Gummi, ein Silikon oder ein thermoplastisches Elastomer ist.

3. Turbine nach einem der vorherigen Ansprüche, **dadurch gekennzeichnet, dass** der zweite Abschnitt (9b) in Röhrenform (10) mit dem ersten Abschnitt (9a) in Form eines Ringes oder eines Halbringes verbunden wird.

4. Turbine nach einem der vorherigen Ansprüche, **dadurch gekennzeichnet, dass** der erste Abschnitt (9a) in Form eines Ringes oder eines Halbringes und der zweite Abschnitt (9b) in Röhrenform (10) aus einem einzigen Teil gebildet werden.

5. Turbine nach einem der vorherigen Ansprüche, **dadurch gekennzeichnet, dass** der erste Abschnitt (9a) in Form eines Ringes oder eines Halbringes und der zweite Abschnitt (9b) in Röhrenform (10) ein einziges Teil bilden, das man durch Formguss erhält.

6. Turbine nach einem der vorherigen Ansprüche, **dadurch gekennzeichnet, dass** der untere Spiralgehäuseabschnitt (3) und der obere Spiralgehäuseabschnitt (4) ausgebildet sind, um zumindest einen Abschnitt einer Abfuhrleitung (7) zu definieren, die dazu dient, das Gas abzuführen, das durch das Flügelrad (2) innerhalb der Kammer gefördert wird, in der das Flügelrad (2) enthalten ist, wobei sich der zweite Abschnitt (9b) in Röhrenform (10) in der Flucht der Abfuhrleitung (7) für Gas positioniert.

7. Turbine nach einem der vorherigen Ansprüche, **dadurch gekennzeichnet, dass** die Struktur (9) aus Elastomer-Material, der untere Spiralgehäuseabschnitt (3) und der obere Spiralgehäuseabschnitt (4) fest miteinander verbunden sind.

8. Turbine nach einem der vorherigen Ansprüche, **dadurch gekennzeichnet, dass** der untere Spiralgehäuseabschnitt (3) und der obere Spiralgehäuseabschnitt (4) aus Polymer-Material, und insbesondere aus einem thermoplastischen sind.

9. Turbine nach einem der vorherigen Ansprüche, **dadurch gekennzeichnet, dass** der untere Spiralgehäuseabschnitt (3) und der obere Spiralgehäuseabschnitt (4) aus einem thermoplastischen Material sind.

10. Turbine nach einem der vorherigen Ansprüche, **dadurch gekennzeichnet, dass** der Motor ermöglicht, das Flügelrad (2) mit einer Geschwindigkeit zwischen 0 und 100.000 U./Min., typischerweise zwischen 10.000 und 60.000 U./Min. anzutreiben.

11. Gerät zur Atmungsunterstützung, eine Turbine nach einem der vorherigen Ansprüche umfassend.

## Claims

1. Turbine for a breathing assistance apparatus, comprising:
- an electric motor (1) interacting with a vaned rotor (2) to generate a gas flow, and
- a volute (3, 4) comprising a volute lower part (3) and a volute upper part (4) having a gas inlet passage (5), coupled to each other and shaped in such a way as to form an inner compartment in which the vaned rotor (2) is arranged,
and in which the volute lower part (3) comprises a first peripheral rim (3a) and the volute upper part (4) comprises a second peripheral rim (4a), said first peripheral rim (3a) and second peripheral rim (4a) facing one another when the volute lower part (3) is coupled to the volute upper part (4),
**characterised in that** at least one structure (9) made from an elastomer material is arranged between the wall around at least one section of the peripheral rim (3a, 4a) of the two volute parts (3, 4), the structure (9) made from elastomer material comprising a first portion (9a) having annular or semi-annular shape, and a second portion (9b) having tubular shape (10) comprising a central passage (11).

2. Turbine according to the preceding claim, **characterised in that** the elastomer material is a rubber, a silicone or a thermoplastic elastomer.

3. Turbine according to any of the preceding claims, **characterised in that** the second portion (9b) having tubular shape (10) connects to the first portion (9a) having annular or semi-annular shape.

4. Turbine according to any of the preceding claims, **characterised in that** the first portion (9a) having annular or semi-annular shape, and the second portion (9b) having tubular shape (10) are formed as a single piece.

5. Turbine according to any of the preceding claims, **characterised in that** the first portion (9a) having annular or semi-annular shape, and the second portion (9b) having tubular shape (10) form a single piece obtained by moulding.

6. Turbine according to any of the preceding claims, **characterised in that** the volute lower part (3) and the volute upper part (4) are shaped to define at least one vent duct (7) used to vent the gas discharged by the vaned rotor (2) within the compartment comprising said vaned rotor (2), the second portion (9b) having tubular shape (10) being positioned in continuation with said gas vent duct (7).

7. Turbine according to any of the preceding claims, **characterised in that** the structure (9) made from elastomer material, the volute lower part (3) and the volute upper part (4) are rigidly attached together.

8. Turbine according to any of the preceding claims, **characterised in that** the volute lower part (3) and that the volute upper part (4) are made from polymer material, in particular from thermoplastic.

9. Turbine according to any of the preceding claims, **characterised in that** the volute lower part (3) and the volute upper part (4) are made from thermoplastic material.

10. Turbine according to any of the preceding claims, **characterised in that** the motor can drive the vaned rotor (2) at a speed between 0 and 100,000 rpm, typically between 10,000 and 60,000 rpm.

11. Respiratory assistance apparatus comprising a turbine according to any of the preceding claims.
